# EUROPEAN PATENT SPECIFICATION

(11) **EP 0 770 678 B1**
(45) Date of publication and mention of the grant of the patent: **22.01.2003**
(21) Application number: 96307692.2
(22) Date of filing: 24.10.1996
(51) Int. Cl.: C12N 9/16, C12N 15/55, C12Q 1/42

(54) **Thermostable alkaline phosphatase of Rhodothermus marinus**
Thermostabile, alkalische Phosphatase aus Rhodothermus Marinus
Phosphatase alcaline thermostable de rhodothermus marinus

(30) Priority: 27.10.1995 US 5965
(43) Date of publication of application: 02.05.1997
(73) Proprietor: Amersham Biosciences Corp., Piscataway, N.J. 08855-1327 (US)
(72) Inventor: Davis, Maria, Twinsburg, Ohio 14087 (US); Szasz, Joseph, Chardon, Ohio 44024 (US)
(74) Representative: Rollins, Anthony John

(56) References cited:
- WO-A-93/08275
- WO-A-95/30756
- WO-A-96/39526
- ANNALES DE BIOLOGIE CLINIQUE, vol. 49, 1991, pages 287-90, XP000653973 TOMAZIC-ALLEN S.J.: "RECOMBINANT BACTERIAL ALKALINE PHOSPHATASE AS AN IMMUNODIAGNOSTIC ENZYME"
- BIOCHIMICA ET BIOPHYSICA ACTA, vol. 526, 1978, pages 457-467, XP000653838 SCHAFFEL S.D. AND HULETT F.M.: "ALKALINE PHOSPHATASE FROM BACILLUS LICHENIFORMIS"
- THE JOURNAL OF BIOLOGICAL CHEMISTRY, vol. 251, 1976, pages 3134-3139, XP000653876 MIN-FUNG YEH AND J.M. TRELA: "PURIFICATION AND CHARACTERIZATION OF A REPRESSIBLE ALKALINE PHOSPHATASE FROM THERMUS AQUATICUS"
- THE JOURNAL OF GENERAL MICROBIOLOGY, vol. 134, 1988, pages 299-306, XP000652195 ALFREDSON G.A. ET AL.: "RHODOTHERMUS MARINUS, GEN. NOV., SP. NOV., A THERMOPHILIC, HALOPHILIC BACTERIUM FROM SUBMARINE HOT SPRINGS IN ICELAND"
- EUROPEAN JOURNAL OF BIOCHEMISTRY, vol. 224, 1994, pages 923-930, XP000652194 SPILLIAERT R. ET AL.: "CLONING AND SEQUENCING OF A RHODOTHERMUS MARINUS GENE, bglA, CODING FOR A THERMOSTABLE BETA-GLUCANASE AND ITS EXPRESSION IN ESCHERICHIA COLI"

### SEQUENCE LISTING

(1) GENERAL INFORMATION:
   (i) APPLICANT: AMERSHAM LIFE SCIENCE, INC. 26111 Miles Road Cleveland, OH 44128 USA
   (ii) TITLE OF INVENTION: THERMOSTABLE ALKALINE PHOSPHATASE OF RHODOTHERMUS MARINUS
   (iii) NUMBER OF SEQUENCES: 4
   (iv) CORRESPONDENCE ADDRESS:
      (A) ADDRESSEE: Amersham International
      (B) STREET: White Lion Road
      (C) CITY: Amersham
      (D) COUNTY: Buckinghamshire
      (E) COUNTRY: England
      (F) Post Code: HP7 9LL
   (v) COMPUTER READABLE FORM:
      (A) MEDIUM TYPE: 3.5" Diskette, 1.44 Mb storage
      (B) COMPUTER: IBM Compatible
      (C) OPERATING SYSTEM: IBM P.C. DOS 5.0
      (D) SOFTWARE: Word Perfect 5.1
   (vi) CURRENT APPLICATION DATA:
      (A) APPLICATION NUMBER: To Be Assigned
      (B) FILING DATE: Herewith
      (C) CLASSIFICATION:
   (vii) PRIOR APPLICATION DATA:
      (A) APPLICATION NUMBER: USSN 60/005,965
      (B) FILING DATE: 27 October 1995
   (viii) ATTORNEY/AGENT INFORMATION:
      (A) NAME: Rollins, Anthony J.
      (B) REFERENCE/DOCKET NUMBER: PB 9512
   (ix) TELECOMMUNICATION INFORMATION:
      (A) TELEPHONE: -1494 543090
      (B) TELEFAX: -1494 543821
(2) INFORMATION FOR SEQ ID NO: 1:
   (i) SEQUENCE CHARACTERISTICS:
      (A) LENGTH: 2936 base pairs
      (B) TYPE: nucleic acid
      (C) STRANDEDNESS: single
      (D) TOPOLOGY: linear
   (xi) SEQUENCE DESCRIPTION: SEQ ID NO: 1:
   GGGTACCGGA GCGGCCGGGG TTGATGAGCG TGGCGAAGCG CTCGACGATC CGGCCGCCCT    60
   GCACACGCAC GGCTGCCAGC TCGATGATCC GATCGGCAGG CCCGGAGCCC GTCGTCTCGG   120
   TATCGACGAC GACGAACGAA ACGGCGTCGA GCTGCATCCC CTCAGTGGGC TTTCGCCGAC   180
   GGCTTCCTCC CGCCGGAGGC CGTCTTCGGT CAGGGCTGGC AGGTCGAAAC CCATGAGCTT   240
   CGGCCAGTAA GCTGACCGTA GTTCAGTCAT TCTCGAAGCT GCCCACCAGC CGATTCGGCG   300
   GCACGGATCC GAAGGCGTAC AGATTGACGT CCACAGCGGT GTGGCCATTC GAGAGTCCAG   360
   CCCACCACGG ACCCGGCGAC CGATCAGCTC GGTAACCACC TCGGCCCAGG TGTCGGGTTC   420
   GGCCGTGGCC TGCGCGACGA GCGCCTGCTC GTCGGCGCGC AGGCTGTCGA GCCCGAGCCA   480
   GGCCTGCAGG AGCGAGTCGG GTCGCTCCGA ACGGCGCAGT GCCGGGATAA GCCGTTCGTA   540
   GGAAGCCTGC ACGCGGGCCA GCACTTCCGG GTGCCAGTCG TAGACGCCGC GGCCGTCCAC   600
   GTTGCGCCCC AGCGACAGAC CGCCCGTCTC GTGGTCGGCC ACCGAGACGA CGAGCGTCTG   660
   TCCGTCGCGG CGGGCAAAGT CGAGCGCCAC GGCCACGGCT TCATCGTAAG CCAGCACTTC   720
   GCGGACGTGG GCGGCGGCGT CGTTGGCATG TCCGGCGTGG TCGATGCGGC TGCCCTCCAC   780
   CATCAGGAAG AAGCCGTCCG GATCGTCCGC CAGCAGCTCG AGTGCCGCGC GGGTCATCTC   840
   GGCCAGCGTA GGGGACCTCT TACCGGGTAC GACCGGTCAA TCTCGTAGGG CAGATGGCTT   900
   GGCCCGAACA GTCCCAGCAC CGGTCTCCTC ACCCCCCGGC GGAAGTCGGC GGCCGTACGC   960
   ACTACCTGGT AGCCCATCGC TTCGGCTTCG CGCAGCAGAT TGCGGCCGTC CTGTCTCCGT  1020
   CCGCCCTCCG CGGTCGGCAG GAAATAGCTC CAGCCACCCC CCAGCAGCAC ATCGACGCGT  1080
   TGCGCCAGCA TCTGCGCGGC GATTTCGCTT TCCATGCGCG CTGCGGCACA TGGGCGGCGA  1140
   AGGCGGCCGG CGTGGCGTGT GAGATCCGGC TGGTCGCCAC CAGCCCGGTC GCCATCCCAC  1200
   GCGCTTTTGC CGCTTCGAGC AACGTGGCGA GTGGGCGTCC GGCCGTATCG ACGGAATCGC  1260
   GCCGTTGTAG GTCTTGACGC CGCAGGCATA GGCCGTCGCC CCGGCGAGCC GGAGTCGGTC  1320
   ACCCGGCTGG AGGCCGAAGC AGTACGCACG GCACCGGTCT GAATGGCATC CAGCGTCAGT  1380
   TCCTCACGTC CCAGCACGGC CCGGGCATAG TCACGGGCCA TCGTGATGCT GGCCGGACCA  1440
   CACCCGTCGG CAATCATCAG GATCAGGTTC TTCGGACGCG GCGACTGGGC CCGAGCCCCT  1500
   TCTCCGACCC CCAACAGCAT CACCAAAAAA CAGCAATATC CAGCGCATTG TCGATTCGCT  1560
   CCCATCTTGA TGAACACGGG CTGTTCAAAG ATACGACAGA TCGGCTTTCA TCCACAGCGC  1620
   CCGGATTATC TACGGAAAGA GCACCATAAA AAAGCCAACC ACCCGAACAC CTGTCCACCT  1680
   TGAGGGCCAA CCCGGCCGGG TTGCGACCTC AACGCAGCAG GCCAAACAGG CCGCACAATC  1740
   ACCCCGATCT GTCCGCCAGA AAATAAACAT CCAGCGCACC AGACTTGCAT ACCGCCGCTT  1800
   AGCATCACTT TCACCTCGGC AATCCGGCTA TCAAGCTTCG CCTCCACCTC GGCAATCCGG  1860
   CTGTCGAGCT TCGCCTCCAC TTCGGCGATC CGGCCGTCGA GCTTCGCTTC AACCTTGGAG  1920
   ATCCGGCCAT TAAACTTTGC CTCTACCTCG GCAATTCGGC GGTCAAGCTT CACCTCTACG  1980
   GCAGCGATGC GTTGTTCCAG ATGCGACACC TCCTACGCTG AATCCGACGG TCCACGCTTC   2040
   GCCACCTCCT CCGTGATCGC GTCTGCTCCA GCACGCGCTT CCACCTCGGT GATGCGATTG   2100
   TCCAGGCGCT TTTCGGTCTC GGCGACCCGC CGCGCGAAAC GCTCCTCTAC AATCCCCAGC   2160
   AGATTGTTAC GCTCGTGATG GGCCGCCTCG TTGAGCAAAT TGATGAGCGC CTCGACACCT   2220
   TCGTCGCCGA GCTTTTCACG CAAAGCTTTT CACGCAAAAC TTTCGGACGG TCAGATTGCC   2280
   ATGAGCGCCT CCACATAGGA TAGGCTAAAA GGAAATCGCA CCTTATTTCT GGAGGTTCCC   2340
   GTTTTCGTCC AAACCGTCCG GTGTCTCGTT GTCAATTGCC CGGCCAGGTT CGGAGGCTAG   2400
   ATTCGAGCTG TCGGGCTAGC CAGGCGCCGT ATTGCGGATT GGGCAGGACG ATCCAGCGCG   2460
   TGCCCCACCA GGAGCGATAA CGCCGGACCA GTGCTCGCCG TGCTTCGGCT CTGGTTTCAG   2520
   GATCGACATA AAGTCGCCCA GCTGGTCGCC GATCTGGAGC AGGATGCGAT AGCGCTGGCC   2580
   CAGGATACCC GGCGCGGTTC TTTGTCGGAA GATCCGCATT CGGGCCGCTC GCCACGCGTC   2640
   AGGATTACGT CGAGCGTGTA CCGGCAGTGG GAAGCCGACG GCCTGCAGGT TACGACGGGT   2700
   GGCCTCCTCC AGGTCGGCCG TGCGGTTGGT CACATAAAAG ACCTGACGCC GTGCCGGCGC   2760
   GCCTCCTGTA CGAACACGAC AGCACCGGGC ACAGGTTCGG CCTGTGCAGC CTGCACCCAG   2820
   CGCGCCCAGC TCTCCGGCGC AAAAGTCCGG CCCGTCGCAA CGAGCCAGGC CTGATAGGGG   2880
   CTGTTGTCGA GCACGGTCTC GTCCACGTCC ACGATCCACT AGTTCTAGAG CGGCCG       2936
(2) INFORMATION FOR SEQ ID NO: 2:
   (i) SEQUENCE CHARACTERISTICS:
      (A) LENGTH: 4454 base pairs
      (B) TYPE: nucleic acid
      (C) STRANDEDNESS: single
      (D) TOPOLOGY: linear
   (xi) SEQUENCE DESCRIPTION: SEQ ID NO: 2:
   TCGCGCGTTT CGGTGATGAC GGTGAAAACC TCTGACACAT GCAGCTCCCG GAGACGGTCA 60
   CAGCTTGTCT GTAAGCGGAT GCCGGGAGCA GACAAGCCCG TCAGGGCGCG TCAGCGGGTG 120
   TTGGCGGGTG TCGGGGCTGG CTTAACTATG CGGCATCAGA GCAGATTGTA CTGAGAGTGC 180
   ACCATATGCG GTGTGAAATA CCGCACAGAT GCGTAAGGAG AAAATACCGC ATCAGGCGCC 240
   ATTCGCCATT CAGGCTGCGC AACTGTTGGG AAGGGCGATC GGTGCGGGCC TCTTCGCTAT 300
   TACGCCAGCT GGCGAAAGGG GGATGTGCTG CAAGGCGATT AAGTTGGGTA ACGCCAGGGT 360
   TTTCCCAGTC ACGACGTTGT AAAACGACGG CCAGTGAATT CGAGCTCGGT ACCGAGCGGC 420
   CGGGGTTGAT GAGCGTGGCG AAGCGCTCGA CGATCCGGCC GCCCTGCACA CGCACGGCTG 480
   CCAGCTCGAT GATCCGATCG GCAGGCCCGG AGCCCGTCGT CTCGGTATCG ACGACGACGA 540
   ACGAAACGGC GTCGAGCTGC ATCCCCTCAG TGGCTTTCGC CGACGGCTTC CTCCCGCCGG 600
   AGCGTCTCGG TCAGGGCTGG CAGGTCGAAA CCCATGAGTT CGGCCAGTAA GCGGCCGAGT 660
   TCGTCATTCT CGAAGCTGCC CACCAGCCGT TCGGCGCCGG GTCCGAAGGC GTACAGATTG 720
   ACGTCCACAG CGGTGTGGCC ATTCGAGGTC CAGCCCACCA CGGCCCGGCG ACCGATCAGC 780
   TCGGTAACCA CCTCGGCCCA GGTGTCGGGT TCGGCCGTGG CCTGCGCGAC GAGCGCCTGC 840
   TCGTCGGCGC GCAGGCTGTC GAGCCCGAGC CAGGCCTGCA GGAGCGAGTC GGGTCGCTCC 900
   GAACGGCGCA GTGCCGGGAT AAGCCGTTCG TAGGAAGCCT GCACGCGGGC CAGCACTTCC 960
   GGGTGCCAGT CGTAGACGCC GCGGCCGTTC ACGTTGCGCC CCAGCGACAG ACCGCCCGTC 1020
   TCGTGGTCGG CCACCGAGAC GACGAGCGTC TGTCCGTCGC GGCGGGCAAA GTCGAGCGCC 1080
   ACGGCCACGG CTTCATCGTA AGCCAGCACT TCGCGGACGT GGGCGGCGGC GTCGTTGGCA 1140
   TGTCCGGCGT GGTCGATGCG GCTGCCCTCC ACCATCAGGA AGAAGCCGTC CGGATCGTCC 1200
   GCCAGCAGCT CGAGTGCCGC GCGGGTCATC TCGGCCAGCG AGGGGACCTC TTCCGGGTCG 1260
   CGGTCAATCT CGTAGGGCAG ATGGCTTGGC CCGAACAGTC CCAGCACCGG TCTCCTCACC 1320
   CCCCGGCGGA AGTCGGCGGC CGTACGCACT ACCTGGTAGC CCATCGCTTC GGCTTCGCGC 1380
   AGCAGATTGC GGCCGTCCTG TCTCCGTCCG CCCTCCGCGG TCGGCAGGAA ATAGCTCCAG 1440
   CCACCCCCCA GCAGCACATG GACGCGTTGC GCCAGCATCT GCGCGGCGAT TTCGCTTTCC 1500
   ATGGCGCGCT GCGGCACATG GGCGGCGAAG GCGGCCGGCG TGGCGTGTGA GATCCGGCTG 1560
   GTCGCCACCA GCCCGGTCGC CATCCCACGC GCTTTTGCCG CTTCGAGCAA CGTGGCGAGT 1620
   GGGCGTCCGG CCGTATCGAC GGCAATCGCG CCGTTGTAGG TCTTGACGCC GCAGGCATAG 1680
   GCCGTCGCCC CGGCGGCCGA GTCGGTCACC CGGCTGGAGG CCGAAGCAGT ACGCACGGCA 1740
   CCGGTCTGAA TGGCATCCAG CGTCAGTTCC TCACGTCCCA GCACGGCCCG GGCATAGTCA 1800
   CGGGCCATCG TGATGCTGGC CGGACCACAC CCGTCGGCAA TCATCAGGAT CAGGTTCTTC 1860
   GGACGCGGCG ACTGGGCCCG AGCCCCTTCT CCGACCCCCA ACAGCATCAC CAAAAACAGC 1920
   AATATCCAGC GCATTGTGCA TCGCTCCATC TGATGAACAC GGGCTGTTCA AAGATACGAC 1980
   AGATCGGCTT TCATCCACAG CCCCGGATAC TACGGAAAGA GCACCATAAA AAAGCCAACC 2040
   ACCCGAACAC CTGTCCACCT TGAGGGCCAA CCCGGCCGGG TTGCGACCTC AACGCAGCAG 2100
   CGCAAACAGC GCCACAATCA CCCCGATCTG TCCCGCCCAG AAAATAAACA TCCAGCGCAC 2160
   CAGACTTGCA TACCGCTCGC TTAGCATCAC TTTCACCTCG GCAATCCGGC TATCAAGCTT 2220
   GGCGTAATCA TGGTCATAGC TGTTTCCTGT GTGAAATTGT TATCCGCTCA CAATTCCACA 2280
   CAACATACGA GCCGGAAGCA TAAAGTGTAA AGCCTGGGGT GCCTAATGAG TGAGCTAACT 2340
   CACATTAATT GCGTTGCGCT CACTGCCCGC TTTCCAGTCG GGAAACCTGT CGTGCCAGCT   2400
   GCATTAATGA ATCGGCCAAC GCGCGGGGAG AGGCGGTTTG CGTATTGGGC GCTCTTCCGC   2460
   TTCCTCGCTC ACTGACTCGC TGCGCTCGGT CGTTCGGCTG CGGCGAGCGG TATCAGCTCA   2520
   CTCAAAGGCG GTAATACGGT TATCCACAGA ATCAGGGGAT AACGCAGGAA AGAACATGTG   2580
   AGCAAAAGGC CAGCAAAAGG CCAGGAACCG TAAAAAGGCC GCGTTGCTGG CGTTTTTCCA   2640
   TAGGCTCCGC CCCCCTGACG AGCATCACAA AAATCGACGC TCAAGTCAGA GGTGGCGAAA   2700
   CCCGACAGGA CTATAAAGAT ACCAGGCGTT TCCCCCTGGA AGCTCCCTCG TGCGCTCTCC   2760
   TGTTCCGACC CTGCCGCTTA CCGGATACCT GTCCGCCTTT CTCCCTTCGG GAAGCGTGGC   2820
   GCTTTCTCAA TGCTCACGCT GTAGGTATCT CAGTTCGGTG TAGGTCGTTC GCTCCAAGCT   2880
   GGGCTGTGTG CACGAACCCC CCGTTCAGCC CGACCGCTGC GCCTTATCCG GTAACTATCG   2940
   TCTTGAGTCC AACCCGGTAA GACACGACTT ATCGCCACTG GCAGCAGCCA CTGGTAACAG   3000
   GATTAGCAGA GCGAGGTATG TAGGCGGTGC TACAGAGTTC TTGAAGTGGT GGCCTAACTA   3060
   CGGCTACACT AGAAGGACAG TATTTGGTAT CTGCGCTCTG CTGAAGCCAG TTACCTTCGG   3120
   AAAAAGAGTT GGTAGCTCTT GATCCGGCAA ACAAACCACC GCTGGTAGCG GTGGTTTTTT   3180
   TGTTTGCAAG CAGCAGATTA CGCGCAGAAA AAAAGGATCT CAAGAAGATC CTTTGATCTT   3240
   TTCTACGGGG TCTGACGCTC AGTGGAACGA AAACTCACGT TAAGGGATTT TGGTCATGAG   3300
   ATTATCAAAA AGGATCTTCA CCTAGATCCT TTTAAATTAA AAATGAAGTT TTAAATCAAT   3360
   CTAAAGTATA TATGAGTAAA CTTGGTCTGA CAGTTACCAA TGCTTAATCA GTGAGGCACC   3420
   TATCTCAGCG ATCTGTCTAT TTCGTTCATC CATAGTTGCC TGACTCCCCG TCGTGTAGAT   3480
   AACTACGATA CGGGAGGGCT TACCATCTGG CCCCAGTGCT GCAATGATAC CGCGAGACCC   3540
   ACGCTCACCG GCTCCAGATT TATCAGCAAT AAACCAGCCA GCCGGAAGGG CCGAGCGCAG   3600
   AAGTGGTCCT GCAACTTTAT CCGCCTCCAT CCAGTCTATT AATTGTTGCC GGGAAGCTAG   3660
   AGTAAGTAGT TCGCCAGTTA ATAGTTTGCG CAACGTTGTT GCCATTGCTA CAGGCATCGT   3720
   GGTGTCACGC TCGTCGTTTG GTATGGCTTC ATTCAGCTCC GGTTCCCAAC GATCAAGGCG   3780
   AGTTACATGA TCCCCCATGT TGTGCAAAAA AGCGGTTAGC TCCTTCGGTC CTCCGATCGT   3840
   TGTCAGAAGT AAGTTGGCCG CAGTGTTATC ACTCATGGTT ATGGCAGCAC TGCATAATTC   3900
   TCTTACTGTC ATGCCATCCG TAAGATGCTT TTCTGTGACT GGTGAGTACT CAACCAAGTC   3960
   ATTCTGAGAA TAGTGTATGC GGCGACCGAG TTGCTCTTGC CCGGCGTCAA TACGGGATAA   4020
   TACCGCGCCA CATAGCAGAA CTTTAAAAGT GCTCATCATT GGAAAACGTT CTTCGGGGCG   4080
   AAAACTCTCA AGGATCTTAC CGCTGTTGAG ATCCAGTTCG ATGTAACCCA CTCGTGCACC   4140
   CAACTGATCT TCAGCATCTT TTACTTTCAC CAGCGTTTCT GGGTGAGCAA AAACAGGAAG   4200
   GCAAAATGCC GCAAAAAAGG GAATAAGGGC GACACGGAAA TGTTGAATAC TCATACTCTT   4260
   CCTTTTTCAA TATTATTGAA GCATTTATCA GGGTTATTGT CTCATGAGCG GATACATATT   4320
   TGAATGTATT TAGAAAAATA AACAAATAGG GGTTCCGCGC ACATTTCCCC GAAAAGTGCC   4380
   ACCTGACGTC TAAGAAACCA TTATTATCAT GACATTAACC TATAAAAATA GGCGTATCAC   4440
   GAGGCCCTTT CGTC                                                      4454
(2) INFORMATION FOR SEQ ID NO: 3:
   (i) SEQUENCE CHARACTERISTICS:
      (A) LENGTH: 1368 base pairs
      (B) TYPE: nucleic acid
      (C) STRANDEDNESS: single
      (D) TOPOLOGY: linear
   (xi) SEQUENCE DESCRIPTION: SEQ ID NO: 3:
   ATGCGCTGGA TATTGCTGTT TTTGGTGATG CTGTTGGGGG TCGGAGAAGG GGCTCGGGCC     60
   CAGTCGCCGC GTCCGAAGAA CCTGATCCTG ATGATTGCCG ACGGGTGTGG TCCGGCCAGC    120
   ATCACGATGG CCCGTGACTA TGCCCGGGCC GTGCTGGGAC GTGAGGAACT GACGCTGGAT    180
   GCCATTCAGA CCGGTGCCGT GCGTACTGCT TCGGCCTCCA GCCGGGTGAC CGACTCGGCC    240
   GCCGGGGCGA CGGCCTATGC CTGCGGCGTC AAGACCTACA ACGGCGCGAT TGCCGTCGAT    300
   ACGGCCGGAC GCCCACTCGC CACGTTGCTC GAAGCGGCAA AAGCGCGTGG GATGGCGACC    360
   GGGCTGGTGG CGACCAGCCG GATCTCACAC GCCACGCCGG CCGCCTTCGC CGCCCATGTG    420
   CCGCAGCGCG CCATGGAAAG CGAAATCGCC GCGCAGATGC TGGCGCAACG CGTCCATGTG    480
   CTGCTGGGGG GTGGCTGGAG CTATTTCCTG CCGACCGCGG AGGGCGGACG GAGACAGGAC    540
   GGCCGCAATC TGCTGCGCGA AGCCGAAGCG ATGGGCTACC AGGTAGTGCG TACGGCCGCC    600
   GACTTCCGCC GGGGGGTGAG GAGACCGGTG CTGGGACTGT TCGGGCCAAG CCATCTGCCC    660
   TACGAGATTG ACCGCGACCC GGAAGAGGTC CCCTCGCTGG CCGAGATGAC CCGCGCGGCA    720
   CTCGAGCTGC TGGCGGACGA TCCGGACGGC TTCTTCCTGA TGGTGGAGGG CAGCCGCATC    780
   GACCACGCCG GACATGCCAA CGACGCCGCC GCCCACGTCC GCGAAGTGCT GGCTTACGAT    840
   GAAGCCGTGG CCGTGGCGCT CGACTTTGCC CGCCGCGACG GACAGACGCT CGTCGTCTCG    900
   GTGGCCGACC ACGAGACGGG CGGTCTGTCG CTGGGGCGCA ACGTGAACGG CCGCGGCGTC    960
   TACGACTGGC ACCCGGAAGT GCTGGCCCGC GTGCAGGCTT CCTACGAACG GCTTATCCCG   1020
   GCACTGCGCC GTTCGGAGCG ACCCGACTCG CTCCTGCAGG CCTGGCTCGG GCTCGACAGC   1080
   CTGCGCGCCG ACGAGCAGGC GCTCGTCGCG CAGGCCACGG CCGAACCCGA CACCTGGGCC   1140
   GAGGTGGTTA CCGAGCTGAT CGGTCGCCGG GCCGTGGTGG GCTGGACCTC GAATGGCCAC   1200
   ACCGCTGTGG ACGTCAATCT GTACGCCTTC GGACCCGGCG CCGAACGGCT GGTGGGCAGC   1260
   TTCGAGAATG ACGAACTCGG CCGCTTACTG GCCGAACTCA TGGGTTTCGA CCTGCCAGCC   1320
   CTGACCGAGA CGCTCCGGCG GGAGGAAGCC GTCGGCGAAA GCCACTGA                1368
(2) INFORMATION FOR SEQ ID NO: 4:
   (i) SEQUENCE CHARACTERISTICS:
      (A) LENGTH: 455 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 4:
   Met Arg Trp Ile Leu Leu Phe Leu Val Met Leu Leu Gly Val Gly Glu
    1               5                  10                  15
   Gly Ala Arg Ala Gln Ser Pro Arg Pro Lys Asn Leu Ile Leu Met Ile
               20                  25                  30
   Ala Asp Gly Cys Gly Pro Ala Ser Ile Thr Met Ala Arg Asp Tyr Ala
           35                  40                  45
   Arg Ala Val Leu Gly Arg Glu Glu Leu Thr Leu Asp Ala Ile Gln Thr
       50                  55                  60
   Gly Ala Val Arg Thr Ala Ser Ala Ser Ser Arg Val Thr Asp Ser Ala
   65                  70                  75                  80
   Ala Gly Ala Thr Ala Tyr Ala Cys Gly Val Lys Thr Tyr Asn Gly Ala
                   85                  90                  95
   Ile Ala Val Asp Thr Ala Gly Arg Pro Leu Ala Thr Leu Leu Glu Ala
               100                 105                 110
   Ala Lys Ala Arg Gly Met Ala Thr Gly Leu Val Ala Thr Ser Arg Ile
           115                 120                 125
   Ser His Ala Thr Pro Ala Ala Phe Ala Ala His Val Pro Gln Arg Ala
       130                 135                 140
   Met Glu Ser Glu Ile Ala Ala Gln Met Leu Ala Gln Arg Val His Val
   145                 150                 155                 160
   Leu Leu Gly Gly Gly Trp Ser Tyr Phe Leu Pro Thr Ala Glu Gly Gly
                   165                 170                 175
   Arg Arg Gln Asp Gly Arg Asn Leu Leu Arg Glu Ala Glu Ala Met Gly
               180                 185                 190
   Tyr Gln Val Val Arg Thr Ala Ala Asp Phe Arg Arg Gly Val Arg Arg
           195                 200                 205
   Pro Val Leu Gly Leu Phe Gly Pro Ser His Leu Pro Tyr Glu Ile Asp
       210                 215                 220
   Arg Asp Pro Glu Glu Val Pro Ser Leu Ala Glu Met Thr Arg Ala Ala
   225                 230                 235                 240
   Leu Glu Leu Leu Ala Asp Asp Pro Asp Gly Phe Phe Leu Met Val Glu
                   245                 250                 255
   Gly Ser Arg Ile Asp His Ala Gly His Ala Asn Asp Ala Ala Ala His
               260                 265                 270
   Val Arg Glu Val Leu Ala Tyr Asp Glu Ala Val Ala Val Ala Leu Asp
           275                 280                 285
   Phe Ala Arg Arg Asp Gly Gln Thr Leu Val Val Ser Val Ala Asp His
       290                 295                 300
   Glu Thr Gly Gly Leu Ser Leu Gly Arg Asn Val Asn Gly Arg Gly Val
   305                 310                 315                 320
   Tyr Asp Trp His Pro Glu Val Leu Ala Arg Val Gln Ala Ser Tyr Glu
                   325                 330                 335
   Arg Leu Ile Pro Ala Leu Arg Arg Ser Glu Arg Pro Asp Ser Leu Leu
               340                 345                 350
   Gln Ala Trp Leu Gly Leu Asp Ser Leu Arg Ala Asp Glu Gln Ala Leu
           355                 360                 365
   Val Ala Gln Ala Thr Ala Glu Pro Asp Thr Trp Ala Glu Val Val Thr
       370                 375                 380
   Glu Leu Ile Gly Arg Arg Ala Val Val Gly Trp Thr Ser Asn Gly His
   385                 390                 395                 400
   Thr Ala Val Asp Val Asn Leu Tyr Ala Phe Gly Pro Gly Ala Glu Arg
                   405                 410                 415
   Leu Val Gly Ser Phe Glu Asn Asp Glu Leu Gly Arg Leu Leu Ala Glu
               420                 425                 430
   Leu Met Gly Phe Asp Leu Pro Ala Leu Thr Glu Thr Leu Arg Arg Glu
           435                 440                 445
   Glu Ala Val Gly Glu Ser His
       450                 455

## Description

### Background of the Invention

Alkaline phosphatases are commonly used in routine biochemical procedures to remove phosphate groups from the termini of nucleic acid molecules. For example, calf intestinal alkaline phosphatase is a heat labile enzyme which is used to remove such phosphate groups, and then is inactivated by exposure to a high temperature. This thermal instability is advantageous because the alkaline phosphatase need not be removed from the reaction mixture prior to subsequent manipulations.

Alkaline phosphatase is also used as a non-radioactive marker for the detection of specific protein or DNA targets. It is conjugated to proteins or DNA oligonucleotides to aid in detection of such targets. Enzyme thermostability is desirable for use in labelling DNA larger than 50bp, and may be useful in other applications for increased stability of the enzyme during storage.

Alkaline phosphatases from various thermophilic and other organisms are known: Yeh and Trela (1976) "Purification and Characterization of a Repressible Alkaline Phosphatase from *Thermus aquaticus"* J. Biol. Chem. **251**:3134-3139; Hartog and Daniel (1992) "An Alkaline Phosphatase from *Thermus* sp Strain Rt41A" Int. J. Biochem. **24**:1657-1660; Schaffel and Hulett (1978) "Alkaline Phosphatase from *Bacillus licheniformis"* Biochimica et Biophysica Acta **526**:457-467; Hulett-Cowling and Campbell (1971) "Purification and Properties of an Alkaline Phosphatase of *Bacillus licheniformis"* Biochem. **10**: 1364-1371.

### Summary of the Invention

Applicant has isolated and purified a novel alkaline phosphatase from the thermophilic species Rhodothermus marinus. This enzyme has a high pH optimum (∼10.8) and is thermostable, retaining 60% of its activity even after 24 hours incubation at 65°C. This enzyme is also tolerant of other denaturing conditions, including overnight incubation in 6M urea at 65°C. The higher pH optimum of this enzyme is a significant advantage. This high pH optimum, and thus stability at high pH, enhances the use of the enzyme in non-radioactive detection systems, for example, when the enzyme is used with streptavidin. In addition, the high pH optimum of the enzyme makes it suitable for use with dioxetane substrates which undergo rapid conversion to the luminescent form at alkaline pH.

The thermostability of the alkaline phosphatase is also advantageous in that following direct cross-linking of the enzyme to nucleic acid probes, it allows hybridization and subsequent washes of such labelled probes under stringent conditions, that is, at elevated temperatures without significant loss of enzyme activity.

Thus, in a first aspect the invention features an enzymatically active portion of the thermostable alkaline phosphatase present in Rhodothermus marinus having a pH optimum greater than 10.5, e.g. 10.8, which is also resistant to a temperature of at least 65°C (i.e., maintains at least 10% of its activity at this temperature).

By phosphatase is simply meant a protein or fragment thereof having an activity which removes a phosphate group from a molecule, such as a DNA molecule or another molecule, such as p-nitrophenyl phosphate (pNPP). An "alkaline phosphatase" is one which is active at a pH greater than 7, and in the present invention has a pH optimum greater than 10.0 using the conditions listed below. Those skilled in the art recognize that pH optimum is dependant on buffer conditions and substrate concentration.

By "thermostable" is meant that the enzyme maintains at least 10% of its activity after heating at 65°C for one hour or longer, preferably for 5 or 10 hours.

While applicant provides one example of an alkaline phosphatase of the present invention, those in the art armed with the fact that an alkaline phosphatase having a pH optimum of about 10.8 exists in nature and can be isolated can now readily screen cellular extracts to determine the presence of such an activity, and can use standard methodology as described herein to isolate and purify such an enzymatic portion.

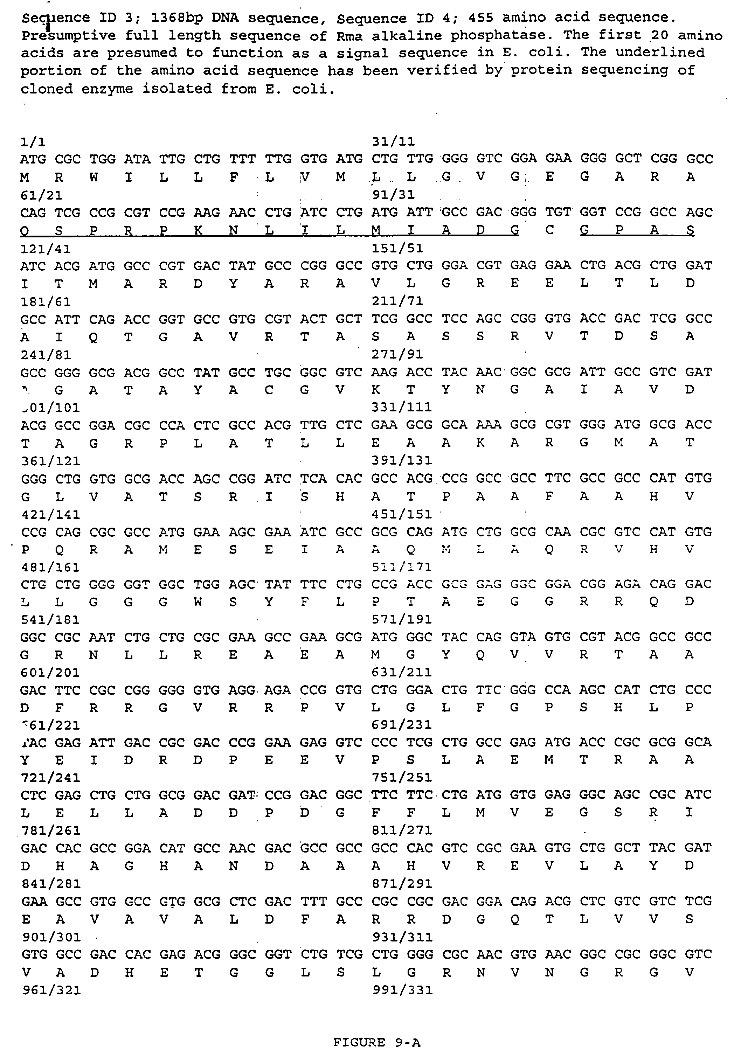

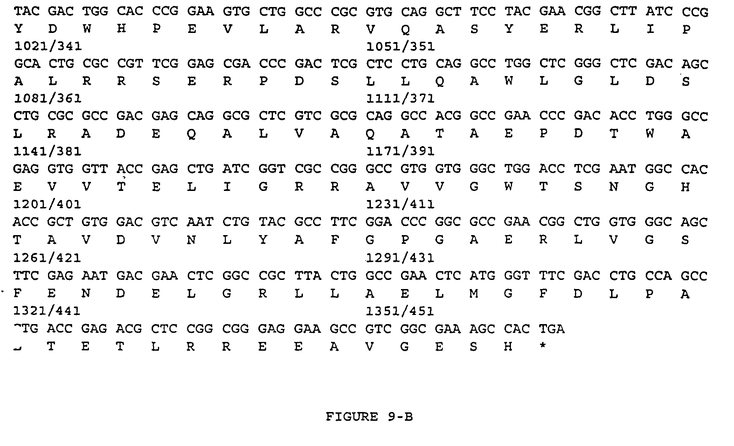

The invention also encompasses other enzymatically active thermostable alkaline phosphatases which have at least 75% homology to the enzymatically active portion of the alkaline phosphatase of Rhodothermus marinus shown in Figure 9 (SEQ. ID. NO. 4). By 75% homology is meant that the thermostable alkaline phosphatase can have an amino acid sequence which differs from that of Rhodothermus marinus by as much as 25 % and still maintain substantially the same enzymatic activity (i.e., the ability to remove a phosphate group from a molecule) as the enzymatically active portion of the alkaline phosphatase of Rhodothermus marinus. Such differences include conservative changes, additions, deletions, and substitutions other than degenerate codons. In preferred embodiments, the amino acid sequence of the alkaline phosphatase includes less than 10 conservative amino acid changes or less than 10 additional amino acids compared to the enzymatically active portion of the alkaline phosphatase of Rhodothermus marinus. By substantially the same enzymatic efficiency is meant at least 50% of the activity of the enzymatically active portion of the thermostable alkaline phosphatase of Rhodothermus marinus

In the present invention, the enzyme is preferably provided in a purified form, that is, it is isolated from the environment in which it naturally occurs. Generally, such an environment is within a bacterial cell and the protein is isolated from the cell wall and/or membranes of that cell such that it is enriched at least 10- or 100-fold compared to its presence in the cell. More preferably, it is enriched 1000- or 10,000- or more fold such that it is an essentially homogeneous preparation, that is, it is the predominant species of protein in a preparation. Even more preferably, the protein is the only species, that is, it represents at least 95% of the proteinaceous material in a sample. Such a protein may be prepared from the bacterial cells in which it naturally occurs, or may be prepared using standard recombinant DNA methodology to cause high levels of expression of the protein in a bacterium or other cell in which it does not naturally occur, e.g., E. coli. A crude extract of such recombinant protein is included within the definition of purified protein.

Using standard techniques the enzyme described below can be readily cloned, for example, by microsequencing of the protein or fragments thereof, preparation of oligonucleotides useful as probes for a library of clones generated from the nucleic acid of a desired organism, e.g., Rhodothermus marinus and screening of that library with such probes to isolate fragments of DNA encoding the protein. Alternatively, an antibody to the protein may be produced and an expression library screened to determine which clone expresses an antigenic determinant recognized by that antibody. Other standard techniques are well known to those of ordinary skill in the art to isolate such genes encoding the claimed proteins. Such genes encode recombinant alkaline phosphatase.

Thus, in a second aspect the invention features recombinant alkaline phosphatase having the above properties, and cells encoding nucleic acid including such recombinant DNA. Equivalent genes encoding such phosphatases can be cloned using standard methodology.

In a third aspect, the invention features a method for use of the above enzymes in labelling of protein or nucleic acid, and in various molecular biology techniques. Thus, the enzymes of the present invention may be used in standard labelling reactions and in diagnostic assays. They may be also used in molecular biology techniques in which removal of a phosphate group is desired.

Other features and advantages of the invention will be apparent from the following description of the preferred embodiments thereof, and from the claims.

### Description of the Preferred Embodiment

The drawings will first briefly be described.

### Drawings

Fig. 1 is a reproduction of a SDS-PAGE gel of purified Rhodothermus marinus alkaline phosphatase. Lane 1 represents molecular weight markers. Lanes 2, 3, 4, and 5 represent 0.4, 0.8, 1.6 and 3.2 ug of purified protein, respectively.

Fig. 2 is a graphical representation of the temperature activity profile of the enzyme. Temperature is presented on the x-axis. Specific activity (u/mg) is presented on the y-axis.

Fig. 3 is a graphical representation of the stability of the enzyme at 65°C. Incubation at 65°C (hours) is presented on the x-axis. Percent initial activity is presented on the y-axis.

Fig. 4 is a graphical representation of the pH activity profile of the enzyme. Open squares represent enzyme activity assayed in 0.1M CAPS, 1mM MgCl₂ and 6mM pNPP. Solid diamonds represent enzyme activity assayed in 0.1M each of ethanolamine, diethanolamine, triethanolamine, 1mM MgCl₂ and 6mM pNPP. On the x-axis pH is presented. Specific activity (u/mg) is presented on the y-axis.

Fig. 5 is a representation of non-isotopic detection of various amounts (0.01 ng, 0.05 ng, 0.1 ng, 0.5 ng, 1 ng, 5 ng, 10 ng) of dot blotted DNA with Rhodothermus marinus alkaline phosphatase.

Fig. 6 represents a Southern Blot of human genomic DNA probed with an N-ras DNA fragment labelled with cloned *Rhodothermus marinus* alkaline phosphatase. Four amounts of total DNA were loaded: 1.0, 0.5, 0.2, 0.1µg. Target DNA is equivalent to 0.5, 0.25, 0.1, 0.05 pg.

Fig. 7 represents a sequence of genomic DNA from *Rhodothermus marinus,* containing the gene for alkaline phosphatase and flanking regions (SEQ. ID. NO. 1).

Fig. 8 represents a sequence of plasmid pCRM1.8, containing a 1.8kb segment of genomic DNA containing alkaline phosphatase and flanking sequences from *Rhodothermus marinus,* cloned into pUC19 (SEQ. ID. NO. 2).

Fig. 9 represents a presumptive full-length protein sequence (455 amino acids) of Rma alkaline phosphatase (SEQ. ID. NO. 4) and the corresponding nucleic acid sequence (1368 bp) (SEQ. ID. NO. 3). The first 20 amino acids are presumed to function as a signal sequence in E. coli. The underlined portion of the amino acid sequence has been verified by protein sequencing of cloned enzyme isolated from E. coli.

### Example: Alkaline Phosphatase from Rhodothermus Marinus

The following is an example of Rhodothermus marinus alkaline phosphatase isolation and characterization. This is not limiting in the invention.

### Culture conditions:

Rhodothermus marinus, strain ATCC 43812 was grown under aerobic conditions at 65°C as described by Alfredsson et. al.(1988) *"Rhodothermus marinus,* gen. nov., sp. nov., a Thermophilic, Halophilic Bacterium from Submarine Hot Springs in Iceland" J. Gen. Microbiology **134**, 299-306. Cells were harvested by continual flow centrifugation and stored at -80°C.

### Activity assay:

Alkaline phosphatase activity was monitored spectrophotometrically at 405nm by following the conversion of p-nitrophenylphosphate (pNPP) to p-nitrophenol. One unit is defined as the amount of enzyme required to release one micromole of p-nitrophenol in one minute. All assays were performed in 100mM CAPS (3-(cyclohexylamino)-1-propanesulfonic acid) pH10, 15% glycerol, 1mm MgCl₂ and 6mM pNPP at 37°C unless otherwise specified.

### Purification:

Frozen cells (33g) were resuspended in 25mM Tris-HCl pH7.4, 50mM NaCl, 1mM MgCl₂ and 0.1% Triton X-100 (100ml final volume) and lysed by sonication. The lysate was cleared of cellular debris by centrifugation, supplemented to a final NaCl concentration of 350mM and chromatographed on DE 52 anionic exchange resin (Whatman; 4g resin per g cells) equilibrated in 25mM Tris-HCl pH 7.4 and 350mM NaCl. The majority of alkaline phosphatase activity appeared in the flow through which was diluted in 25mM Tris-HCl pH 7.4 to lower the salt concentration to 100mM. The diluted flow through was applied to a column of Heparin Sepharose CL-6B resin (Pharmacia) equilibrated in 25mM Tris-HCl pH7.4 and 100mM NaCl. Alkaline phosphatase activity did not bind to the resin. The flow through was next applied to a Q-Sepharose FF column (Pharmacia) equilibrated in 25mM Tris-HCl pH 7.4 and 100mM Nacl. The column was developed with a linear gradient from 100 to 350mM NaCl in 25mM Tris-HCl pH 7.4. Alkaline phosphatase eluted at ∼ 250mM NaCl. Appropriate fractions were pooled, supplemented to 1mM MgCl₂ and resolved on hydroxylapatite (BIO-RAD) equilibrated in 25mM Tris-HCl pH 7.4 and 10mM NaCl (Buffer A). The enzyme activity was eluted with a linear gradient from 100% Buffer A to 100% Buffer B (50mM Na phosphate pH 7.0). Fractions containing alkaline phosphatase activity were pooled, equilibrated into 50mM Tris-HCl pH 9.3 and 25mM NaCl (Buffer A) on Centriprep-30 concentrators (Amicon) and applied to a Q-Sepharose FF column equilibrated in Buffer A. The enzymatic activity was eluted with a linear gradient from 25 to 600mM NaCl in Buffer A. Pooled fractions were exchanged into 25mM Tris-HCl pH 7.4 and 25mM NaCl as described above and applied to a DEAE-Sephacel column (Pharmacia) in the same buffer. Alkaline phosphatase was eluted with a linear gradient from 25 to 300mM NaCl in 25mM Tris-HCl. Appropriate DEAE-Sephacel fractions were combined, exchanged into 25mM Tris-HCL, 25mM NaCl and 50mM MgCl₂ on a Centriprep 30 apparatus and heated at 100°C for 10 minutes. Insoluble, heat-denatured proteins were removed by centrifugation.

### Characterization:

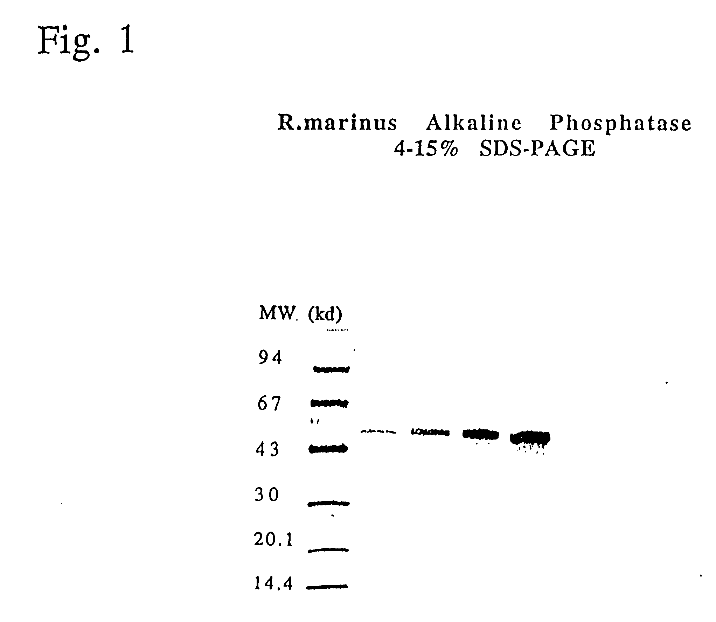

Following the above purification procedure analysis of the preparation by SDS-PAGE revealed that enzyme subunits corresponded to two closely spaced polypeptides which migrated at an apparent molecular weight of approximately 55,000 daltons (Fig. 1). The gel used was purchased from BioRad as a Mini-Protein II Ready Gel, Catalogue No. 161-0902, 4-15% gradient gel and used according to the manufacturer's specifications. The presence of two protein bands, both of which display enzyme activity, may represent isozymes of the same protein, a post-translationally modified form of the enzyme, a partial proteolytic cleavage or possibly an artifact of the purification procedure. If the latter is true, then those skilled in the art recognize that the enzyme may be purified by other procedures which may or may not yield two polypeptides.

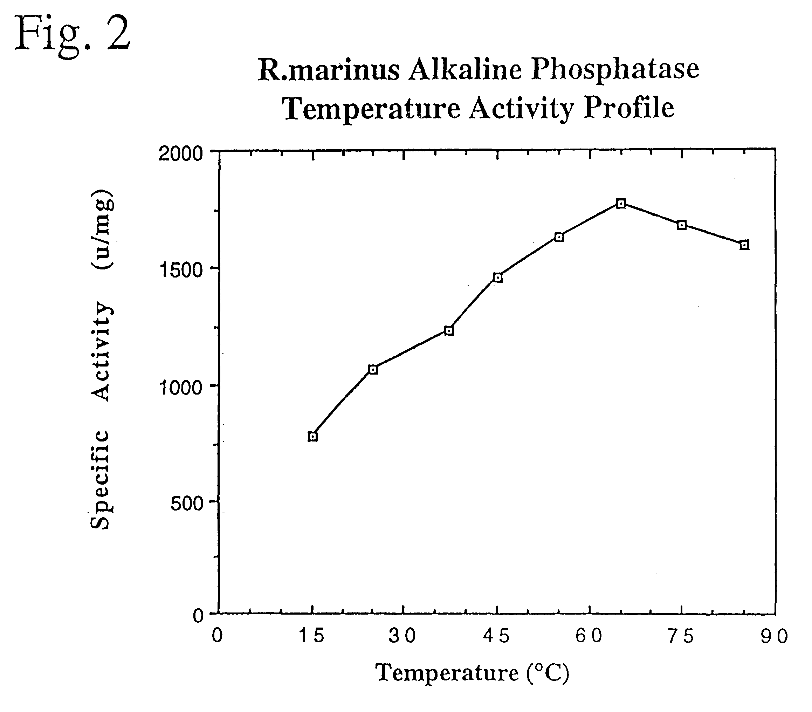

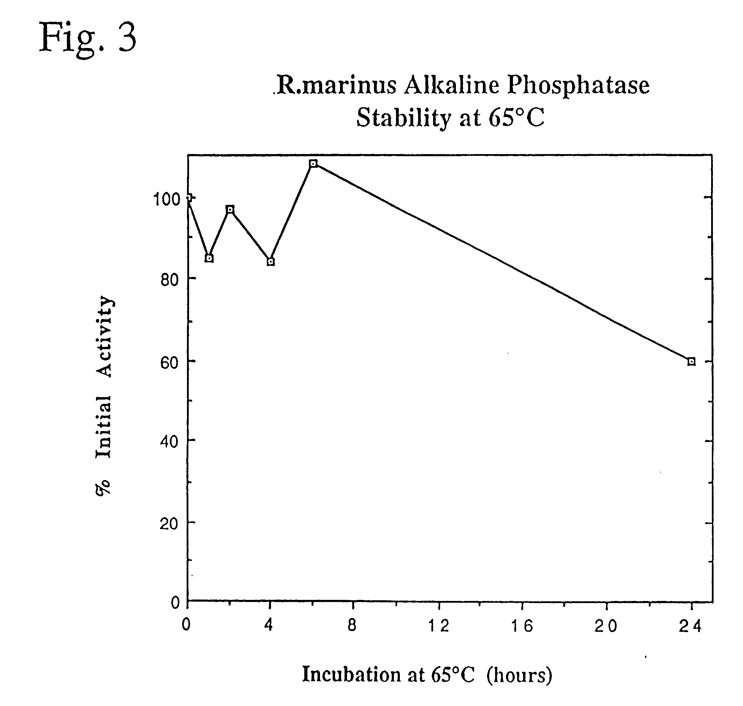

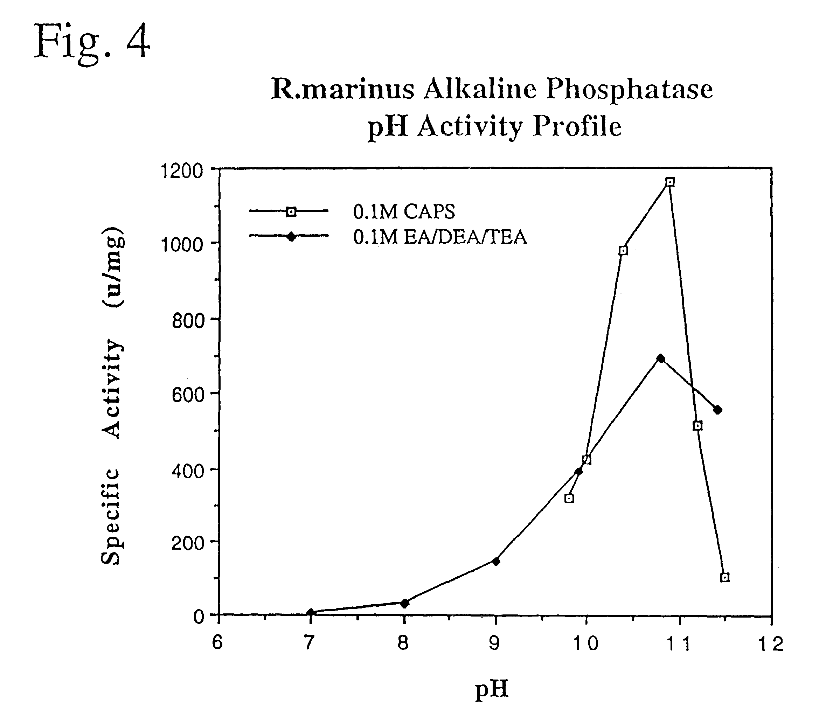

The final product represented a greater than 350-fold purification of the crude cell lysate as determined by specific activity studies. The enzyme preparation was further characterized by determining the temperature and pH of optimal enzyme activity. To determine the temperature of optimal enzyme activity, stock alkaline phosphatase (0.32 mg/ml) was diluted 250-fold in 25mM Tris-HCl pH7.4, 25mM NaCl and 1mM MgCl₂ and assayed for activity under standard conditions (see above) at the indicated temperatures. Results are presented in Fig. 2. To determine the pH of optimal enzyme activity, stock alkaline phosphatase was diluted 200-fold in 25mM Tris-HCl pH 7.4, 25mM Nacl and 1mM MgCl. Enzyme assays were performed (as above) at the indicated pH values at 37°C in either 0.1M CAPS, 1mM MgCl₂ and 6mM pNPP or 0.1M each of ethanolamine, diethanolamine, triethanolamine, 1mM MgCl₂ and 6mM pNPP. Results are presented in Fig. 4. To determine enzyme stability at 65°C, stock alkaline phosphatase was diluted 20-fold in 25mM Tris-HCl pH 7.4, 25mM NaCl, 1mM MgCl₂ and 15% glycerol and incubated at 65°C for the indicated times. Samples were diluted an additional 10-fold prior to activity assays performed under standard conditions. Results are presented in Fig. 3.

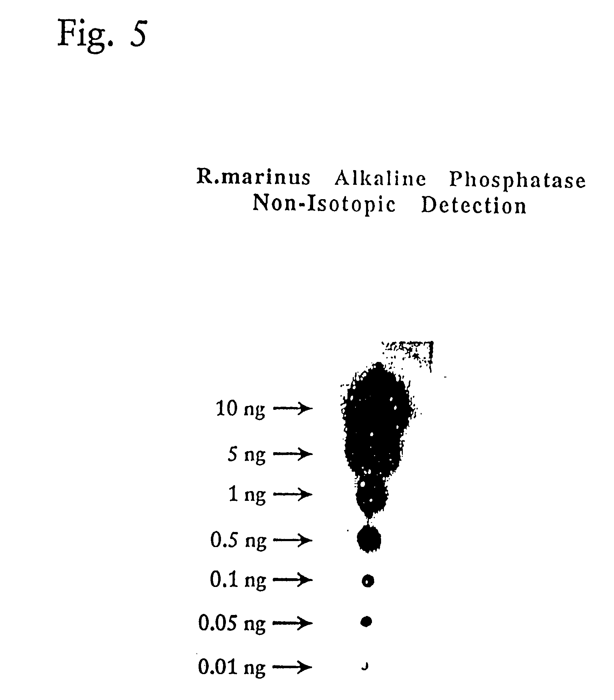

The alkaline phosphatase was also used in the non-isotopic detection of dot-blotted DNA (Fig. 5). Target DNA (λ-Hind III) was denatured in TE + 0.2M NaOH and spotted in the various amounts (0.01 ng, 0.05 ng, 0.1 ng, 0.5 ng, 1 ng, 5 ng, 10 ng) onto a nylon membrane (Biodyne A; Pall) and fixed by baking for 1 hour at 80°C. The membrane was prehybridized for 1.5 to 2 hours at 65°C in ECL Gold hybridization buffer (Amersham) supplemented with 400mM NaCl, 4% casein and 50mM MglC₂. Probe was prepared by crosslinking R. marinus alkaline phosphatase to λ-Hind III DNA. Equal volumes (typically 10ul each) of heat denatured probe (10ng/ul in 10mM Na phosphate pH 5.5), alkaline phosphatase (60ng/ml in 10mM Na phosphate pH 5.5) and glutaraldehyde (1.5%) were mixed and incubated at 37° for 45 minutes. The enzyme/probe complex was added directly to the prehybridization reaction (10ng probe/ml hybridization buffer) and allowed to hybridize overnight at 65°C. Following hybridization, the membrane was washed as follows: 2X SSC, 25mM Tris pH 7.4, 10mM MgCl₂, 0.1% SDS (3 x 5 minutes at room temperature); 0.2X SSC, 25mM Tris pH 7.4, 10mM MgCl₂ (3 x 5 minutes at 37°C); 0.1 M CAPS pH 10, 15% glycerol, 2mM MgCl₂ (30 minutes at room temperature). After washing the membrane was placed in a seal-a-meal bag with ∼ 20ul CDP-Star substrate (Tropix) per cm² membrane and exposed to autoradiography film for 1 minute.

### Cloning of Rhodothermus marinus Alkaline Phosphatase

Genomic DNA was isolated from *Rhodothermus marinus* using standard procedures. Chromosomal DNA was subjected to partial *Sau 3AI* digestion and resolved by gel electrophoresis in low-melt agarose. Fragments ranging from 4-9 kb were isolated from the gel and ligated into pBluescript SK+ (Stratagene) which had been digested with BamHI and dephosphorylated. Following ligation, DNA was transformed into ultracompetent *E. coli* strain XL2-Blue MRF' (Stratagene) and plated on LB agar plates containing 100 ug/ml ampicillin, 50 ug/ml BCIP (5-bromo-4-chloro-3-indolyl phosphate, a chromogenic indicator) and 5mM IPTG (isopropyl-β-D-thiogalactopyranoside). The plates were incubated overnight at 37°C. A single blue colony (pBRM1) was observed out of approximately 6000 transformants.

### Clone pBRM1

Restriction analysis of clone pBRM1 indicated an insert of approximately 6 kb which encoded the alkaline phosphatase activity in cell lysates. No significant enzyme activity was observed in parallel studies performed with empty pBluescript vector. The alkaline phosphatase activity was found to be thermostable, as greater than 75% of enzyme activity was retained after heating for 10 minutes at 100°C. In-gel colorimetric assays performed on cell lysates which had been renatured following SDS-PAGE indicated that the enzymatic activity was localized to a polypeptide which migrated at an apparent molecular weight of ∼46,000 Daltons. pBRM1 was transformed into *E. coli* CC118 [ara D139 Δ(ara, leu) 7697 Δlac X74 phoA Δ20 galE galK thi rpsE rpoB arg Eam recA1] and grown on BCIP indicator plates. This *E. coli* strain contains a deletion in the alkaline phosphatase (pho A) gene. The development of a blue colony indicated that the cloned enzyme is distinct from native *E. coli* alkaline phosphatase (BAP).

### Clone pBRM1/KpnI

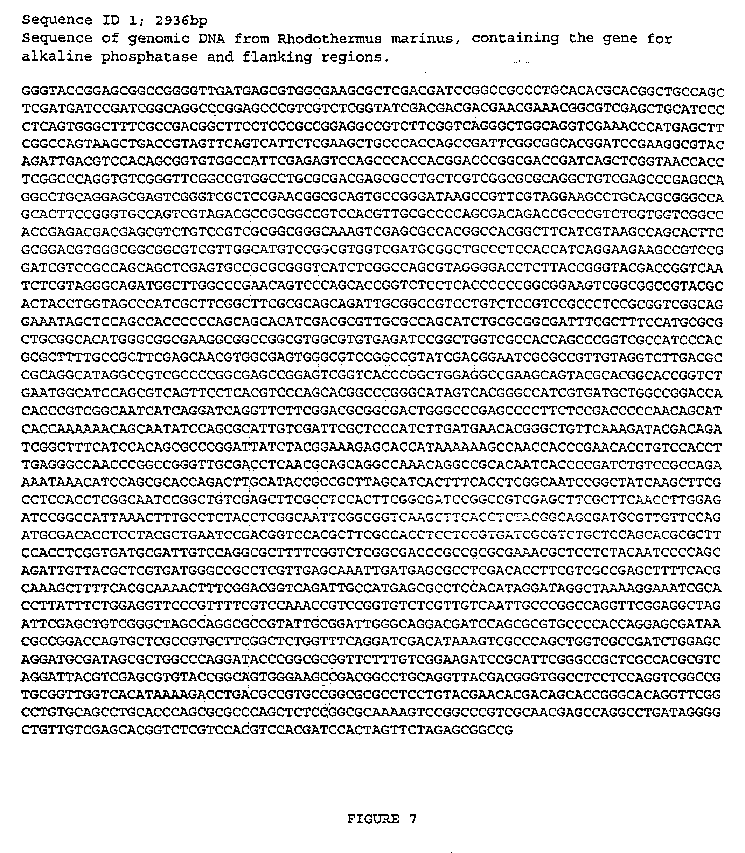

Mapping, deletion and expression studies indicated that approximately one half of the 6 kb insert in pBRM1 could be deleted without affecting enzyme activity. The smaller clone containing an insert of ∼3 kb was designated as pBRM1/KpnI (SEQ ID NO. 1. See Figure 7). To facilitate DNA sequencing, pBRM1/KpnI was digested with *Sac*II to yield 0.45, 1.9 and 0.5 kb fragments which were individually subcloned into pBluescript.

### Clone pCRM1.8

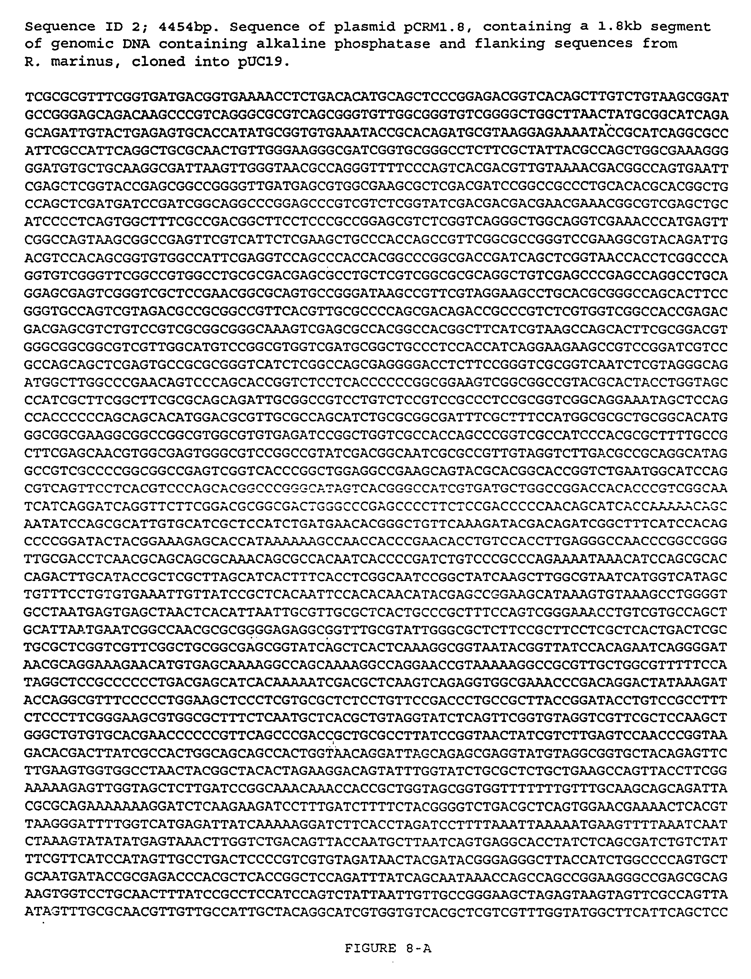

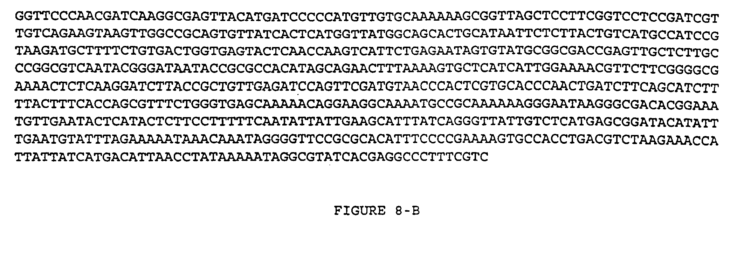

BLAST analysis (Gish et al., (1993) Nat. Genet. 3:266-72) of the DNA sequence data indicated that ∼1 kb of the insert did not code for alkaline phosphatase and thus could be deleted. pBRM1/KpnI was digested with *Hind*III/K*pn*I to release a 1.8kb fragment which was subsequently subcloned into pUC19. The resultant clone was designated as pCRM1.8 (SEQ ID NO. 2. See Figure 8).

### Expression and Purification of Cloned Alkaline Phosphatase

An overnight culture of *E.coli* strain JM109 containing the recombinant plasmid pBRM1.8 was diluted 1:40 in LB + 100 ug/ml ampicillin and grown with shaking at 37°C until the A₆₀₀ reached 1.30. The culture was induced by the addition of 2.5 mM IPTG and allowed to incubate for an additional 18 hours. The cells were pelleted by centrifugation at 6000 X g for 20 minutes at 4°C and stored at -80°C. Frozen cells (12g) were thawed and resuspended in 60 ml of lysis buffer (25 mM Tris,HCL pH 7.4, 50 mM NaCl, 1 mM MgCl_{2,} 1 mM EDTA, 0.05% Triton X-100 and 1 mg/ml lysozyme). The resuspension was stirred occasionally for 45 minutes at 4°C, sonicated to reduce viscosity and clarified by centrifugation at 70,000 X g for 30 minutes at 4°C. The supernatant was supplemented to a final concentration of 50 mM MgCl₂ and heated at 80°C for 20 minutes to precipitate heat labile proteins which were subsequently removed by centrifugation as above. The supernatant was diluted 1:1 with Buffer A (25 mM Tris,HCL pH 7.4, 20 mM NaCl and 1mM MgCl₂) to reduce salt concentration and chromatographed on DE 52 anionic exchange resin (Whatman). The column was developed with a 6-column volume linear gradient from 20 to 500mM NaCl in Buffer A. Alkaline phosphatase activity eluted at ∼180 mM NaCl. Appropriate fractions were pooled and applied to a hydroxylapatite column (Bio-gel HTP; BioRad) equilibrated in 25 mM Tris•HCL pH 7.4 and 20 mM NaCl (Buffer B). The column was washed with two column volumes of Buffer B to remove residual MgCl₂ and then developed with an 8-column volume linear gradient of Buffer A to 150 mM Na phosphate pH 7.0. Alkaline phosphatase eluted at a phosphate concentration of ∼40 mM. Pooled fractions were diluted 1:1 in Buffer B and applied to a Q Sepharose FF anion exchange column (Pharmacia). The column was washed with Buffer B to remove residual phosphate and then developed with a 10-column volume linear gradient of 20 to 500 mM NaCl in Buffer A. Enzyme activity was found to elute at ∼250mM NaCl. The final product was found to be greater than 90% pure as determined by SDS-PAGE with a specific activity of ∼3000 units/mg. The cloned protein has an apparent molecular weight which is less than that of native protein isolated from *R. marinus.* Protein sequencing of the amino terminus of both proteins revealed that the majority of the mature alkaline phosphatase isolated from *R. marinus* begins at serine residue 22 (SEQ ID NO. 4., See Figure 9), whereas the protein isolated from *E.coli* begins at glutamine residue 21. The difference in apparent molecular weight between the two proteins therefore is not due to a truncation at the amino terminus of the cloned protein. Amino acids upstream of these residues are presumed to function as signal peptides, as alkaline phosphatase is a periplasmic enzyme in *E. coli* and other bacteria.

### DNA Probe Labelled with Cloned Rhodothermus marinus alkaline phosphatase

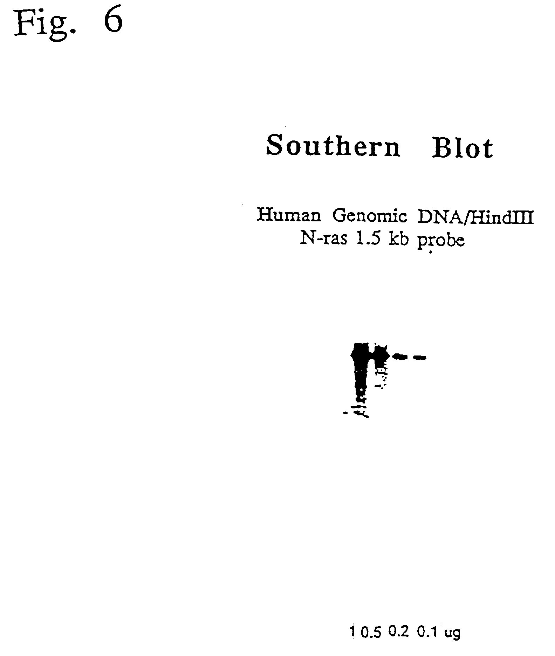

Cloned alkaline phosphatase was used for the non-isotopic detection of human genomic DNA (Fig. 6) The N-ras gene is easily detected in a 0.1 µg sample of total human genomic DNA by Southern blot analysis. The blot was prepared by fractionating a *Hind* III digestion of genomic DNA through a 1.0% agarose gel followed by capillary transfer onto a Hybond N membrane (Amersham) using 10 X SSC buffer. The membrane was prehybridized for 1.5 to 2 hours in modified ECL Gold hybridization buffer (from Amersham, 2M urea in place of 6M urea, 0.2% SDS in place of 0.4% SDS.) supplemented with 400 mM NaCl, 4% casein and 20 mM MgCl₂. The probe was prepared by cross-linking cloned *R. marinus* alkaline phosphatase to N-ras DNA. Equal volumes (typically 10µl each) of heat denatured probe (10ng/ml in water), cloned alkaline phosphatase diluted immediately prior to use (40 ng/ml in 10 mM MES buffer pH 5.0) and formaldehyde (2% in water) were mixed and incubated at 37°C for 30 minutes. The probe was added directly to the prehybridization reaction (5 ng/ml hybridization buffer) and allowed to hybridize overnight at 50°C. Following hybridization, the membrane was washed as follows: one rinse and then 2 x 10 minute washes with the following buffer preheated to 50°C (washing was done on a shaker platform at RT): 100 mM Na phosphate pH 7.0, 150 mM NaCl, 2M urea, 0.4% SDS, 10mM MgCl₂. This was followed by one rinse and then 2 x 5 minute washes with the following buffer preheated to 55°C (wash at RT): 100mM Na phosphate pH 7.0, 150 mM NaCl, 10 mM MgCl₂. The last rinse was done at RT with 50 mM Tris pH 10, 100 mM NaCl, 2 mM Mg Cl₂. The membrane was placed in a sealed plastic bag with 20 µl CDP-star substrate (Tropix) per cm² membrane and exposed to autoradiography film for 2 hours.

### Uses

Alkaline phosphatases of this invention have several potential uses in the numerous non-isotopic methods for the detection of proteins and nucleic acids. For example, the high pH optimum of this enzyme makes it suitable with dioxetane substrates which undergo rapid conversion to the luminescent form at alkaline pH. When using streptavidin conjugated alkaline phosphatase on positively charged membranes, as in nucleic acid hybridization, a pH greater than 9.5 is preferred to give decreased background. In addition, the high thermostability of this alkaline phosphatase makes it useful for direct crosslinking to nucleic acid probes. Hybridization and subsequent washes can be carried out under stringent conditions (i.e., elevated temperatures or in the presence of denaturants such as 6M urea) without loss of enzyme activity.

Alkaline phosphatases from different organisms may (or may not) behave similarly during purification. The high pH optimum for activity cannot be exploited for purification per se, but see below for screening. The high temperature optimum will be useful in purifying such enzymes after cloning into hosts that grow at a moderate temperature, such as E. coli. Extracts from E. coli could be heat treated to precipitate all proteins that denature at elevated temperatures.

If an enzyme is desired which is stable at 65°-75°C, it is possible to enhance the chances of discovery of such an enzyme by trying to isolate novel organisms that grow well at those temperatures. One could also select for organisms that are tolerant of high pH. In addition, knowing that an alkaline phosphatase is desired, one can then screen organisms, or libraries of recombinant clones, for alkaline phosphatase activity by use of the compound 5-bromo-4-chloro-3-indolyl phosphate (BCIP). A blue color is obtained when the phosphate group is removed from this compound, making it very convenient to screen for activity. A pH activity profile would then be prepared to determine whether the phosphate removing activity was an alkaline phosphatase.

Other embodiments are within the following claims.

## Claims

1. A purified enzymatically active thermostable alkaline phosphatase obtainable from Rhodothermus marinus comprising an amino acid sequence with at least 75% homology to the amino acid sequence set out in SEQ ID NO: 4 or to the enzymatically active portion (aa positions 22 to 455) of the sequence set out in SEQ ID NO: 4.

2. The alkaline phosphatase of claim 1, wherein the amino acid sequence includes less than 10 conservative amino acid changes compared to the amino acid sequence set out in SEQ ID NO:4.

3. The alkaline phosphatase of claim 1, wherein the amino acid sequence includes less than 10 additional amino acid changes compared to the amino acid sequence set out in SEQ ID NO:4.

4. The purified thermostable alkaline phosphatase according to claim 1 which has an apparent subunit molecular weight of about 45,000-55,000 as determined by SDS-PAGE and stable to heating at 65°C for 1 hour.

5. A purified recombinant nucleic acid encoding a thermostable alkaline phosphatase of any of claims 1-4.

6. A cell comprising the recombinant nucleic acid of claim 5.

7. Method for detecting nucleic acid or protein in sample comprising the step of providing a nucleic acid, protein, or antibody labelled with a thermostable alkaline phosphatase as claimed in any of claims 1-4.

## Patentansprüche

1. Eine gereinigte, enzymatisch aktive, thermostabile, alkalische Phosphatase erhältlich aus Rhodothermus marinus, umfassend eine Aminosäuresequenz mit mindestens 75% Homologie zu der Aminosäuresequenz aufgeführt in SEQ ID NO:4 oder zu dem enzymatisch aktiven Teil (Aminosäurepositionen 22 bis 455) der Sequenz aufgeführt in SEQ ID NO:4.

2. Die alkalische Phosphatase nach Anspruch 1, worin die Aminosäuresequenz im Vergleich zur Aminosäuresequenz aufgeführt in SEQ ID NO:4 weniger als 10 konservative Aminosäureaustausche enthält.

3. Die alkalische Phosphatase nach Anspruch 1, worin die Aminosäuresequenz im Vergleich zur Aminosäuresequenz aufgeführt in SEQ ID NO:4 weniger als 10 zusätzliche Aminosäureaustausche enthält.

4. Die gereinigte, thermostabile, alkalische Phosphatase nach Anspruch 1, welche ein apparentes Untereinheitsmolekulargewicht von etwa 45.000-55.000 besitzt, bestimmt mittels SDS-PAGE, und stabil ist gegenüber Erwärmen bei 65°C über 1 Stunde.

5. Eine gereinigte, rekombinante Nukleinsäure, die für eine thermostabile, alkalische Phosphatase nach mindestens einem der Ansprüche 1-4 kodiert.

6. Eine Zelle, umfassend die rekombinante Nukleinsäure nach Anspruch 5.

7. Ein Verfahren zum Nachweis von Nukleinsäure oder Protein in einer Probe, umfassend den Schritt der Bereitstellung einer Nukleinsäure, eines Proteins oder Antikörpers, markiert mit einer thermostabilen, alkalischen Phosphatase wie beansprucht nach mindestens einem der Ansprüche 1-4.

## Revendications

1. Phosphatase alcaline thermostable purifiée, enzymatiquement active, pouvant être obtenue à partir de *Rhodothermus marinus,* comprenant une séquence d'acides aminés présentant au moins 75 % d'homologie avec la séquence d'acides aminés indiquée en SEQ ID NO : 4 ou avec la portion enzymatiquement active (positions des aa 22 à 455) de la séquence indiquée en SEQ ID NO : 4.

2. Phosphatase alcaline selon la revendication 1, dans laquelle la séquence d'acides aminés comprend moins de 10 changements d'acides aminés conservatifs par rapport à la séquence d'acides aminés indiquée en SEQ ID NO : 4.

3. Phosphatase alcaline selon la revendication 1, dans laquelle la séquence d'acides aminés comprend moins de 10 changements d'acides aminés additionnels par rapport à la séquence d'acides aminés indiquée en SEQ ID NO : 4.

4. Phosphatase alcaline thermostable purifiée selon la revendication 1 qui a un poids moléculaire apparent de sous-unité d'environ 45 000 à 55 000, comme déterminé par SDS-PAGE et est stable quand elle est chauffée à 65°C pendant 1 heure.

5. Acide nucléique recombinant purifié codant pour une phosphatase alcaline thermostable selon l'une quelconque des revendications 1 à 4.

6. Cellule comprenant l'acide nucléique recombinant selon la revendication 5.

7. Méthode de détection d'un acide nucléique ou d'une protéine dans un échantillon comprenant l'étape consistant à fournir un acide nucléique, une protéine ou un anticorps marqué avec une phosphatase alcaline thermostable telle que revendiquée dans l'une quelconque des revendications 1 à 4.
